Europäisches Patentamt

(19) European Patent Office

Office européen des brevets

(11) EP 0 651 991 B1

## (12) FASCICULE DE BREVET EUROPEEN

(45) Date de publication et mention de la délivrance du brevet:
16.12.1998 Bulletin 1998/51

(51) Int. Cl.⁶: $A61K\ 7/02$, $A61K\ 7/021$

(21) Numéro de dépôt: 94402447.0

(22) Date de dépôt: 28.10.1994

(54) **Composition cosmétique destinée au maquillage de la peau comprenant une fraction pulvérulente**

Pulverischen Anteil enthaltende kosmetische Zusammensetzung zum Hautschminken

Cosmetic composition for skin make-up comprising a powdery fraction

(84) Etats contractants désignés:
AT BE CH DE ES FR GB IT LI NL SE

(30) Priorité: 08.11.1993 FR 9313238

(43) Date de publication de la demande:
10.05.1995 Bulletin 1995/19

(73) Titulaire: L'OREAL
75008 Paris (FR)

(72) Inventeurs:
• Le Bras, Véronique
  F-75002 Paris (FR)
• Gabin, Philippe
  F-91440 Bures-sur-Yvette (FR)

(74) Mandataire:
Peuscet, Jacques et al
SCP Cabinet Peuscet et Autres,
78, avenue Raymond Poincaré
75116 Paris (FR)

(56) Documents cités:
EP-A- 0 447 286          EP-A- 0 502 769
DE-A- 2 540 877          US-A- 4 994 264

• DATABASE WPI Week 8006, Derwent Publications Ltd., London, GB; AN 80-10351C & JP-A-54 163 831 (SHISEIDO KK) 16 Juin 1978
• DATABASE WPI Section Ch, Week 8622, Derwent Publications Ltd., London, GB; Class D08, AN 86-141933 & JP-A-61 078 709 (SHISEIDO KK) 22 Avril 1986
• DATABASE WPI Section Ch, Week 9139, Derwent Publications Ltd., London, GB; Class D08, AN 91-286066 & JP-A-3 190 813 (TOMBOW ENPITSU KK) 20 Août 1991 & PATENT ABSTRACTS OF JAPAN vol. 15, no. 445 (C-0884) 13 Novembre 1991 & JP-A-03 190 813 (TOMBOW PENCIL CO LTD) 20 Août 1991
• D4 = Wuithier, P. (1972). Le pétrole. Raffinage et génie chimique, Tome 1, Editions Technip, p. 5.
• D5 = Perry, R.H.; Green, D.W. (éd.)(1984). Perry's chemical engineers' handbook, McGraw-Hill, p. 3-95.

**Description**

La présente invention concerne une composition cosmétique à forte teneur en charge(s) destinée au maquillage de la peau. L'invention concerne également un procédé de préparation de ladite composition par extrusion dans un mélangeur à vis. L'invention concerne enfin un produit de maquillage mettant en oeuvre ladite composition.

On sait que les compositions cosmétiques contenant une dispersion de poudre(s) sont très largement utilisées pour estomper les défauts de la peau, tels que les micro-reliefs et les rides, et pour estomper les variations de coloration ou conférer à la peau une coloration souhaitée. Les produits de maquillage de ce type se présentent généralement sous forme de fond de teint liquide ou crémeux ou sous forme de pastille compactée. Dans tous les cas, les compositions mises en oeuvre sont constituées de poudres dispersées dans un liant. Les poudres contiennent généralement des pigments colorés, tels que des oxydes de fer, et des charges particulaires, comme le mica, le talc ou la silice. On a, par exemple, décrit, dans le brevet US-A-4 839 163. une composition, dont le liant est une émulsion huile-dans-eau, dans laquelle est dispersée une fraction pulvérulente contenant des charges essentiellement lamellaires, qui confèrent à la composition un bon pouvoir couvrant ; malheureusement, ces charges lamellaires agissent surtout par réflexion de la lumière et présentent, pour cette raison, l'inconvénient de conférer à la peau un aspect brillant peu naturel.

On a donc cherché, tout en conservant le pouvoir couvrant, c'est-à-dire une bonne capacité de masquer les imperfections de la peau, à améliorer la translucidité du maquillage obtenu avec de telles compositions pour donner à la peau maquillée un aspect naturel. Pour ce faire, on a, par exemple, proposé dans la demande de brevet japonais 61-69708 de préparer des compositions dans lesquelles on enrobe les particules de la charge avec une résine acrylique ; l'inconvénient est que la préparation d'une telle composition nécessite un traitement préalable d'enrobage des charges, enrobage dont l'intégrité dans la formulation finale n'est d'ailleurs pas assurée.

On a également proposé dans le brevet français 2 673 372, une composition qui permet de réaliser une couche de maquillage translucide estompant bien les reliefs de la peau et donnant néanmoins à la peau maquillée un aspect naturel ; ce résultat est atteint en dispersant dans un liant huileux, outre des pigments et des poudres lamellaires, une poudre, dont les particules sont sphériques ou sphéroïdales, et en respectant un rapport volumique liant/poudre(s) déterminé. Plus précisément, cette composition comporte, dispersée dans un liant formé par une émulsion comportant une phase huileuse et une phase aqueuse, une fraction pulvérulente constituée d'au moins une poudre et compatible avec l'application sur la peau, la concentration particulaire volumique C de ladite composition étant définie par la formule :

$$C = \frac{V_{CT}}{V_{CT} + V_G}$$

où $V_{CT}$ représente le volume de la fraction pulvérulente totale et $V_G$ représente le volume des huiles non volatiles de la phase huileuse du liant, volume qui est obtenu en déduisant du volume total de la phase grasse, y compris les ingrédients oléosolubles éventuels, celui correspondant, s'il y en a, aux huiles ayant à 25°C une tension de vapeur saturante supérieure à 50 Pa, la valeur critique C* de la concentration C susdéfinie étant celle pour laquelle $V_G$ est égale au volume juste nécessaire pour combler les interstices entre les particules de la fraction pulvérulente, tel que mesuré selon la norme ASTM D 281-84, C étant au moins égal à C*. Dans cette composition, la fraction pulvérulente contient au moins 75 % en poids de particules sphériques ou sphéroïdales et C* est compris entre 3 et 90 %. Cependant, comme il ressort des exemples de ce brevet, la quantité de charge incorporée dans ce type de composition est, en pratique, limitée car, à partir d'un certain pourcentage, la composition devient trop pâteuse pour permettre, au moyen des mélangeurs à turbine utilisés de façon classique pour la réalisation de ces émulsions, une homogénéisation correcte ; l'aspect de l'émulsion devient granuleux et mat, ce qui est le signe d'une composition hétérogène. Or, il est extrêmement intéressant d'augmenter le pourcentage de charge(s) dans une composition de maquillage de ce type, pour améliorer le pouvoir couvrant et l'estompage des reliefs de la peau.

Dans la demande de brevet japonais 54/163831 publiée le 26 Décembre 1979, on a proposé une composition de maquillage anhydre renfermant, dispersée dans un liant gras, une forte proportion d'une fraction pulvérulente constituée d'un mélange de pigments et de poudre de mica. La fabrication de cette composition s'effectue en réalisant, avec de l'eau ou des solvants volatils, un mélange contenant les différents constituants de la composition, en extrudant le mélange obtenu et en faisant sécher le produit extrudé. Malheureusement, les charges dans le produit de maquillage ainsi réalisé sont, en dehors des pigments conférant la coloration, exclusivement constituées par de la poudre de mica, c'est-à-dire une poudre lamellaire, dont il a déjà été indiqué précédemment qu'elle conférait au maquillage un aspect réfléchissant peu naturel qu'il est souhaitable d'éviter.

On a maintenant découvert, selon la présente invention, qu'il était possible d'introduire un fort pourcentage de charges dans une émulsion à condition de respecter une règle définissant le taux de charges en fonction de la densité de la fraction pulvérulente utilisée. Dans le cas des formulations permettant d'estomper les rides, on sait, notamment d'après le brevet français 2 673 372 précité, que la capacité d'estompage est liée au fait que la concentration particulaire volumique C, précédemment définie, est supérieure à la concentration particulaire volumique critique C*, précédemment définie également ; plus le rapport C/C* est grand, plus la capacité d'estompage de la composition est élevée ; cependant on considérait jusqu'à ce jour que la réduction de la proportion de phase grasse, qui correspond à une augmentation de la valeur de C à charges constantes, entraînait une réduction du confort sur la peau et une augmentation de la tendance à l'instabilité de l'émulsion ; en outre, comme il a été précédemment indiqué, l'augmentation du volume de charges à quantité de phase grasse constante, qui correspond aussi à une augmentation de la valeur de C, était plafonnée par l'hétérogénéité de la composition finale. Selon l'invention, on a maintenant constaté que, si l'on se maintient dans une gamme spécifique pour le choix du taux de charges T défini plus loin, on peut obtenir par extrusion une composition pâteuse parfaitement homogène qui, lorsqu'elle est utilisée pour le maquillage de la peau, s'avère posséder un pouvoir couvrant remarquable et permet d'obtenir un maquillage translucide d'aspect parfaitement naturel. En outre, les compositions selon l'invention, bien qu'à fort taux de charges, contiennent des volumes d'huile suffisants pour que la pâte obtenue soit maléable et de mise en forme aisée. On peut donc conditionner cette composition sous forme de bâton (ou "stick") que l'on conserve en conditionnement étanche et qui donne, à l'application sur la peau, l'impression agréable d'une poudre avec sensation de fraîcheur ; on peut aussi conditionner une telle composition sous forme particulaire, par exemple billes, vermicelles ou granulés, par adjonction d'un granulateur en sortie d'extrudeur, ces particules étant mises en suspension dans un gel pour l'application. Il doit être noté que l'inclusion des particules dans un gel est aisée du fait de l'hydrophilie de la composition, qui est une émulsion. A l'application, le gel permet l'étalement des particules et l'utilisateur a une impression de fondant et une sensation de fraîcheur ; après séchage sur la peau, il reste une fine pellicule de poudre qui a un grand pouvoir estompeur de rides.

Les compositions selon l'invention peuvent également contenir des charges dites "incompactables". Par "charges incompactables", on entend une matière première qui, à partir d'un certain pourcentage dans un mélange de poudres, ne permet pas de réaliser un compactage au moyen d'une presse mécanique ou conduit à un compactage non satisfaisant ; on sait, en effet, qu'une tablette compactée doit, en cosmétique, répondre à un certain nombre de critères, notamment le maintien de l'intégrité du compact au cours du temps, l'obtention d'une surface plane pour le compact et la solidité du compact en cas de choc. Parmi les matières dites "incompactables", on trouve des microsphères, des microcapsules et des poudres lamellaires. A titre d'exemple, on peut indiquer que si l'on met en oeuvre comme charge des microsphères creuses en matériau thermoplastique de densité inférieure à 0,1 dans un produit de maquillage compacté, on constate l'apparition d'amorce de fragmentation et de morcellement dès que le pourcentage de ces microsphères dans la composition est supérieur à 1 % en poids environ, cette dégradation étant le résultat de phénomènes de relaxation. La composition pâteuse obtenue selon l'invention peut, de manière surprenante, être compactée par des moyens classiques tels qu'une presse mécanique, même en cas de présence d'une proportion importante de matières premières dites "incompactables" car la cohésion est assurée par la présence d'eau ; la pâte compactée peut ensuite être séchée, à température ambiante ou à l'étuve, pour obtenir une tablette de poudre sèche, qui possède la propriété de contenir des charges réputées "incompactables". La composition pâteuse selon l'invention peut également être mise en forme de façon quelconque, notamment par moulage, puis séchée par des méthodes classiques. On peut donc obtenir de la sorte des bâtons ou des éléments de forme quelconque alors qu'antérieurement les poudres sèches avaient une forme imposée par les récipients de compactage utilisés. Il convient de noter que, parmi les charges "incompactables", se trouvent notamment les microsphères de faible densité, qui procurent un toucher très doux et dont l'introduction dans les poudres compactées était, jusqu'àlors, limitée à de très faibles quantités, ce qui n'est plus le cas pour les compositions selon l'invention.

La présente invention a, en conséquence, pour objet une composition cosmétique destinée au maquillage de la peau comprenant une fraction pulvérulente de densité D compatible avec l'application sur la peau, dispersée dans un liant formé par une émulsion comportant une phase grasse et une phase aqueuse, dans laquelle :

- les particules de ladite fraction ont des dimensions comprises entre 0,5 et 100 µm,
- la concentration particulaire volumique de ladite composition est au moins égale à la concentration particulaire critique, et
- le taux de charges T, défini en % par la formule :

$$T = 100 \times \frac{\text{poids de la fraction pulvérulente hors pigments}}{\text{poids total de la composition}}$$

est compris entre T0 et 1,8 x T0, la valeur T0 étant calculée de la manière suivante :

- si D ≤ 0,1         T0 = 10
- si 0,1 < D ≤ 0,6          T0 = (60 x D) + 4

- si D > 0,6      T0 = 40

Dans la présente description, la concentration particulaire volumique C de la composition est définie par la formule $C = V_{CT}/(V_{CT} + V_G)$ où $V_{CT}$ représente le volume de la fraction pulvérulente totale et $V_G$ représente le volume des constituants non volatils de la phase grasse du liant, volume qui est obtenu en déduisant du volume total de la phase grasse, y compris les ingrédients oléosolubles éventuels, celui correspondant, s'il y en a, aux huiles ayant à 25°C une tension de vapeur saturante supérieure à 50 Pa.

La valeur critique $C^*$ de la concentration C susdéfinie est celle pour laquelle $V_G$ est égal au volume juste nécessaire pour combler les interstices entre les particules de la fraction pulvérulente, tel que mesuré selon la norme ASTM D 281-84.

La fraction pulvérulente peut comprendre toutes poudres connues utilisées de façon usuelle pour la préparation de compositions cosmétiques de maquillage : ces poudres peuvent comporter au moins un pigment et/ou au moins une charge. On choisit, de préférence, une fraction pulvérulente pour laquelle $10\% < C^* < 80\%$ ; en outre, on choisit avantageusement $C > 80\%$.

De plus, si D > 0,6, on choisit avantageusement un taux de charges T compris entre 40 % et 65 %.

Les pigments peuvent être choisis parmi les pigments minéraux, les pigments organiques et les pigments nacrés. Parmi les pigments minéraux, on peut citer, à titre d'exemples :

- le dioxyde de titane (rutile ou anatase), éventuellement traité en surface et codifié dans le "Color Index" (CI) sous la référence CI 77891 ;
- les oxydes de fer noir, jaune, rouge et brun (CI 77499, 77492, 77491) ;
- le violet de manganèse (CI 77742) ;
- le bleu outremer (CI 77007) ;
- l'oxyde de chrome (CI 77288) ;
- l'oxyde de chrome hydraté (CI 77289) et
- le bleu ferrique (CI 77510).

Parmi les pigments organiques, on peut citer, par exemple, les pigments : D & C red n° 19 (CI 45170) ; D & C red n° 9 (CI 15585) ; D & C red n° 21 (CI 45380) ; D & C orange n° 4 (CI 15510) ; D & C orange n° 5 (CI 45370) ; D & C red n° 27 (CI 45410) ; D & C red n° 13 (CI 15630) ; D & C red n° 7 (CI 15850:1) ; D & C red n° 6 (CI 15850:2) ; D &C yellow n° 5 (CI 19140) ; D & C red n° 36 (CI 12085) ; D & C orange n° 10 (CI 45425) ; D & C yellow n° 6 (CI 15985) ; D & C red n° 30 (CI 73360) ; D &C red n° 3 (CI 45430) ; le noir de carbone (CI 77266) et les laques à base de carmin de cochenille (CI 75470) ;

Les pigments nacrés peuvent être choisis notamment parmi :

- les pigments nacrés blancs, tels que le mica recouvert d'oxyde de titane, l'oxychlorure de bismuth ; et
- les pigments nacrés colorés, tel que le mica titane avec des oxydes de fer, le mica titane avec du bleu ferrique ou de l'oxyde de chrome, le mica titane avec un pigment organique du type précité, ainsi que les pigments à base d'oxychlorure de bismuth.

Ces pigments peuvent représenter jusqu'à 30 % en poids par rapport au poids total de la composition.
Les charges sont choisies notamment parmi :

- le talc, qui est un silicate de magnésium hydraté, utilisé sous forme de particules généralement de dimensions inférieures à 40 µm ; le talc possède des propriétés absorbantes de l'humidité et est utilisé surtout en raison de son toucher onctueux ;
- les micas, qui sont des aluminosilicates de compositions variées, qui se présentent sous forme d'écailles ayant des dimensions de 2 à 200 µm, de préférence de 5 à 70 µm et une épaisseur de 0,1 à 5 µm, de préférence de 0,2 à 3 µm ; les micas peuvent être d'origine naturelle (par exemple muscovite, margarite, roscoelithe, lipidolithe, biotite) ou d'origine synthétique ; les micas sont généralement transparents et permettent de conférer à la peau un aspect satiné ;
- l'amidon, en particulier l'amidon de riz ;
- le kaolin, qui est un silicate d'aluminium hydraté, qui se présente sous la forme de particules lamellaires ayant des dimensions généralement inférieures à 30 µm, et qui possède de bonnes propriétés d'absorption des corps gras ;
- les oxydes de zinc et de titane, généralement utilisés sous la forme de particules ayant des dimensions ne dépassant pas quelques micromètres (ou même inférieures à 1 µm dans le cas de l'oxyde de titane) ; ces oxydes ont un toucher onctueux, ont un bon pouvoir couvrant et ont une opacité importante ;
- le carbonate de calcium précipité qui, sous forme de particules de dimensions inférieures à 10 µm, a un toucher onctueux et permet d'obtenir un aspect mat ;

- le carbonate et l'hydrocarbonate de magnésium, qui possèdent notamment des propriétés de fixation des parfums ;
- la silice ;
- des savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium ; ces savons, présents généralement sous la forme de particules ayant des dimensions inférieures à 10 µm, ont un toucher onctueux et facilitent l'adhérence de la poudre sur la peau.

On peut aussi utiliser les poudres de polymères synthétiques non expansées, tels que le polyéthylène, les polyesters (par exemple isophtalate ou téréphtalate de polyéthylène), et les polyamides (par exemple le nylon), sous la forme de particules ayant des dimensions inférieures à 50 µm, qui possèdent des propriétés absorbantes et permettent de conférer à la peau un aspect velouté ; les poudres minérales telles que la silice sphérique ; les dioxydes de titane sphériques comme celui commercialisé sous le nom commercial "SPHERITITAN" ; les billes de verre et de céramique commercialisées par la société "3M" sous les dénominations commerciales "MACROLITES" ; des poudres de matériaux organiques d'origine naturelle comme les amidons de maïs, de blé, de riz, réticulées ou non ; des poudres de polymères de synthèse, réticulées ou non, sphéronisées, comme des poudres de polyamide telles que des poudres de poly-β-alanine et des poudres de nylon comme celles commercialisées par la société "ATOCHEM" sous la dénomination commerciale "ORGASOL", des poudres d'acides polyacrylique ou polymétacrylique, des poudres de polystyrène réticulées par le divinylbenzène, des poudres de résine de silicone, des poudres de téflon telles que celles commercialisées par la société "MONTEFLUOS" sous la dénomination commerciale "FLUON" ou celles commercialisées par la société "HOECHST" sous la dénomination commerciale "HOSTAFLONQ".

Comme il résulte de l'énumération ci-dessus, la fraction pulvérulente peut comprendre au moins une charge dite "incompactable" et c'est là une caractéristique importante de l'invention car, si l'on moule ou compacte la composition selon l'invention et qu'on la fait sécher ensuite, on obtient un produit de maquillage anhydre ayant la forme souhaitée et renfermant un fort taux de charges "incompactables", ce qui était totalement impossible dans l'état de la technique. Sous la dénomination "charges incompactables", on inclut notamment :

a) les microsphères pleines réalisées en tout matériau organique ou inorganique compatible avec une utilisation sur la peau, c'est-à-dire non-irritant et non toxique.

Ces microsphères peuvent être micro-poreuses auquel cas elles ont une surface spécifique d'au moins 0,5 $m^2$/g et, de préférence, d'au moins 1 $m^2$/g, ladite surface spécifique n'ayant pas de limite supérieure autre que celle résultant de la possibilité pratique de réaliser des microsphères de porosité très élevée ; la surface spécifique peut, par exemple, atteindre 1000 $m^2$/g ou même davantage. On peut citer, à titre d'exemples, les microsphères micro-poreuses vendues par la société "DOW CORNING" sous la dénomination commerciale "POLYTRAP" et les microsphères micro-poreuses de la société "SEPPIC" vendues sous la dénomination commerciale de "MICROPEARL M" ou "MICROPEARL M 100". Les microsphères micro-poreuses peuvent être imprégnées ou non, notamment avec des agents actifs : on peut citer, à cet égard, les microsphères "PLASTIC POWDER FPSQ" imprégnées de squalane et les microsphères "SILICA BEADS".

b) les microsphères creuses réalisées en matériau thermoplastique et préparées par des procédés connus, tels que ceux décrits dans US-A-3 615 972 et EP-A-0 56219.

Ces microsphères creuses peuvent notamment être réalisées en polymères ou copolymères de dérivés éthyléniques, tels que le polyéthylène, le polystyrène, le copolymère (chlorure de vinyle/acrylonitrile) ou le polyacrylonitrile, en polyamides, en polyesters, en polymères urée-formaldéhyde ou en copolymères de chlorure de vinylidène, tel que le copolymère (chlorure de vinylidène/acrylonitrile). On peut citer, par exemple, les microsphères creuses commercialisées sous la dénomination commerciale "EXPANCEL" par la société "KEMANORD PLAST" ou sous la dénomination commerciale "MICROPEARL F 80 ED" par la société "MATSUMOTO".

c) les microcapsules réalisées en tout matériau organique ou inorganique compatible avec une utilisation sur la peau, c'est-à-dire non irritant et non toxique.

Ces microcapsules peuvent ou non renfermer un agent actif. Les microcapsules en matériau organique polymère sont réalisées, notamment, en polymères ou copolymères dérivés d'acides, d'amines ou d'esters monomères à insaturation éthylénique, en polymères urée-formaldéhyde, en polymères ou copolymères de chlorure de vinylidène. A titre d'illustrations, on mentionnera les microcapsules faites de polymères ou copolymères d'acrylate ou de métacrylate de méthyle ou encore de copolymères chlorure de vinylidène/ acrylonitrile ; parmi ces derniers, on indiquera, notamment, ceux qui contiennent, en poids, de 20 à 60 % de motifs dérivés de chlorure de vinylidène, de 20 à 60 % en poids de motifs dérivés d'acrylonitrile, et de 0 à 40 % en poids d'autres motifs tels que des motifs dérivés d'un monomère acrylique ou styrénique.

On peut citer, à titre d'exemples, les microcapsules de "MACROLITE" vendues par la société "3 M", les microcapsules "Q-MAX" vendues par la société "Q-MAX" et les microcapsules "3 M" vendues par la société "3 M".

d) certaines charges lamellaires et, notamment, les mica-titanes, certaines séricites, telle que celle vendue par la société "WHITTAKER" sous la dénomination commerciale "SERICITE BC 282", et certains talcs, tel que celui vendu par la société "NIPPON" sous la dénomination commerciale "TALC K 1" ou celui vendu par la société "LUZENAC" sous la dénomination commerciale "EXTRA STEAMIC 00S".

Il convient, en outre, d'indiquer que les pigments et les charges peuvent être enrobés de substances telles que des acides aminés, des silicones, des sels métalliques ou du collagène, notamment afin de modifier leur état de surface.

Dans un mode de réalisation avantageux, l'émulsion qui constitue le liant de la composition selon l'invention comprend une quantité d'eau importante ; on préfère que la composition contienne de 25 à 50 % en poids d'eau et mieux, de 30 à 45 % en poids d'eau. La phase grasse de l'émulsion comprend au moins un constituant gras, liquide ou solide à la température ambiante.

Parmi les constituants gras utilisables, on peut citer, notamment, les huiles ou corps gras, d'origine animale, végétale, minérale ou synthétique, les cires, d'origine animale, végétale, minérale ou synthétique ou leurs mélanges.

Les huiles ou corps gras sont, en particulier, choisis parmi l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba ou l'huile d'arachide ; une huile d'hydrocarbures, telle que les huiles de paraffine, le squalane, la vaseline ; des esters tels que le myristate d'isopropyle, le palmitate d'isopropyle, le stéarate de butyle, le laurate d'hexyle, l'isononate d'isononyle, le palmitate de 2-éthyl-hexyle, le laurate de 2-hexyldécyle, le palmitate de 2-octyl-décyle, le myristate de 2-octyl-dodécyle, le succinate de 2-di-éthyl-héxyle, le malate de diisostéaryle, le lactate de 2-octyl-dodécyle, le triisostéarate de glycérine ou le triisostéarate de diglycérine ; une huile de silicone comme les polyméthylsiloxanes, les polyméthylphénylsiloxanes, des polysiloxanes modifiés par des acides gras, des polysiloxanes modifiés par des alcools gras, des polysiloxanes modifiés par des polyoxyalkylènes, des silicones fluorées ; des huiles perfluorées et/ou organofluorées ; des acides gras supérieurs tels que l'acide myristique, l'acide palmitique, l'acide stéarique, l'acide béhénique, l'acide oléique, l'acide linoléique, l'acide linolénique ou l'acide isostéarique ; des alcools gras supérieurs tels que le cétanol, l'alcool stéarylique ou l'alcool oléique.

Les cires peuvent être choisies dans le groupe formé par les cires animales, les cires végétales, les cires minérales, les cires synthétiques et les fractions diverses de cires naturelles. Parmi les cires animales utilisables, on peut citer les cires d'abeille, les cires de lanoline et les cires d'insectes de Chine. Parmi les cires végétales, on peut citer la cire de carnauba, de candélila, d'ouricurry, les cires de fibres de liège, les cires de canne à sucre, les cires du Japon, les cires de jojoba hydrogénées et les huiles hydrogénées, qui sont obtenues par hydrogénation catalytique de corps gras composés de chaînes grasses, linéaire ou non. en $C_8$-$C_{32}$ et qui ont les qualités correspondant à la définition des cires. On peut citer, en particulier, l'huile de tournesol hydrogénée, l'huile de ricin hydrogénée, l'huile de coprah hydrogénée et la lanoline hydrogénée. Parmi les cires minérales, on peut citer les paraffines, les cires microcristallines, les cires de Montan et les ozokérites. Parmi les cires synthétiques, on peut citer les cires de polyéthylène, les cires obtenues par la synthèse de Fisher et Tropsch, les copolymères cireux ainsi que leurs esters, et les cires de silicone telles que les polyalcoxy et polyalkylsiloxanes.

La phase grasse peut, de façon connue, contenir au moins un actif cosmétique lipophile, ainsi que des ingrédients oléosolubles divers généralement utilisés en cosmétique, tels que, par exemple, des parfums, l'ensemble de ces additifs de la phase grasse pouvant représenter jusqu'à 20 % en poids par rapport au poids total de la phase grasse.

La phase grasse peut comprendre des huiles volatiles ayant à 25° C une tension de vapeur saturante supérieure à 50 pascals. Lorsqu'elles sont présentes, ces huiles représentent, généralement, moins de 10 % en poids par rapport au poids total de la composition et moins de 20 % en poids par rapport au poids de la phase grasse. Les huiles volatiles s'évaporent au contact de la peau lorsque la composition selon l'invention est appliquée sur celle-ci mais elles sont utiles car elles facilitent l'étalement de la composition lors de l'application sur la peau. Parmi les huiles volatiles, on peut citer, par exemple, des huiles de silicone, telles que l'hexaméthyldisiloxane, le cyclopentadiméthylsiloxane, le cyclotétraméthylsiloxane, des huiles fluorées telles que celles vendues par la société "MONTEFLUOS" sous la dénomination commerciale "GALDEN" et des huiles isoparafiniques telles que celles vendues sous la dénomination commerciale "ISOPAR" par la société "ESSO".

La phase aqueuse de l'émulsion, qui constitue le liant de la composition selon l'invention, peut contenir, en plus de l'eau :

- des épaississants, tels que des gommes naturelles, par exemple la gomme arabique, la gomme adragante, la gomme de guar, des dérivés de la cellulose, des pectines, comme les dérivés de l'acide alginique et du carraghen, des bentoniques et des silices colloïdales, des polysaccharides, des macromolécules synthétiques, notamment à groupements vinyliques ou acryliques, des matières amilacées, des dérivés phosphorylés d'alcool aliphatique hydroxylé, des triglycérides naturelles ou semi-synthétiques inter-estérifiées ;
- des humectants ou hydratants, tels que la glycérine et le collagène ;

- des matières premières hydrosolubles utilisées généralement en cosmétique.

Ces additifs de la phase aqueuse peuvent représenter jusqu'à 40 % en poids par rapport au poids total de la composition.

Comme il a été précédemment indiqué, les compositions selon l'invention sont fabriquées par extrusion dans un mélangeur à vis. La présente invention a donc également pour objet un procédé de préparation d'une composition telle que ci-dessus définie, caractérisé par le fait que l'on effectue le mélange des divers constituants de ladite composition dans un mélangeur extrudeur-cuiseur comportant, dans une enveloppe externe munie en sortie d'une filière d'extrusion, un (ou deux) arbre(s) entraîné(s) en rotation de façon que la structure périphérique d'un arbre coopère avec l'enveloppe externe et, le cas échéant, avec la structure périphérique de l'autre arbre pour assurer un mélangeage de la matière et son déplacement dans l'enveloppe externe vers la filière d'extrusion.

Dans un mode préféré de réalisation, l'arbre (ou les arbres) est (sont) constitué(s) d'au moins deux manchons successifs, dont la partie intérieure s'adapte sur un axe entraîné en rotation et la partie extérieure a une structure périphérique différente selon les manchons ; l'arbre (ou chaque arbre) comporte au moins un manchon formant une vis de transport situé du côté de l'alimentation du mélangeur, au moins un manchon à contrefilet et/ou un manchon multilobes, et au moins un manchon formant une vis de transport situé à l'extrémité de sortie du mélangeur. On peut avantageusement prévoir que l'on introduit dans le mélangeur, simultanément mais en des points décalés le long de l'(ou des) arbre(s), la phase grasse du liant, la phase aqueuse du liant et la fraction pulvérulente, la température allant en décroissant depuis la tête d'arbre jusqu'à la filière d'extrusion ; la température peut être d'environ 70-90° C au voisinage de la tête d'arbre et décroître jusqu'à environ 20-30° C au voisinage de la filière d'extrusion. De préférence, les constituants non-thermosensibles ou volatils de la phase grasse sont introduits au voisinage de la tête d'arbre de même que tout ou partie de la fraction pulvérulente, les constituants non-thermosensibles de la phase aqueuse sont introduits en un point plus éloigné de la tête d'arbre et les constituants thermosensibles ou volatils de l'émulsion ainsi que le reste éventuel de la fraction pulvérulente sont introduits en un point encore plus éloigné de la tête d'arbre.

Les mélangeurs extrudeur-cuiseur utilisables selon l'invention sont des appareils de type connu, mis en oeuvre notamment de façon courante dans l'industrie alimentaire et l'industrie chimique. Ces mélangeurs comportent une enveloppe externe munie en sortie d'une filière d'extrusion, enveloppe à l'intérieur de laquelle un (ou deux) arbre(s) est (sont) entraîné(s) en rotation de façon que la structure périphérique d'un arbre coopère avec l'enveloppe externe et, le cas échéant, avec la structure périphérique de l'autre arbre, pour assurer un mélangeage de la matière et son déplacement dans l'enveloppe externe vers la filière d'extrusion. De préférence, l'arbre (ou chacun des arbres) est constitué d'au moins deux manchons successifs, dont la partie intérieure s'adapte sur un axe entraîné en rotation et dont la partie extérieure peut avoir diverses structures périphériques ; parmi les structures classiques, on peut citer, d'une part, un filetage de vis hélicoïdale, dont le pas conduit la matière traitée de l'entrée vers la sortie du mélangeur (désigné plus loin par "DF"), d'autre part, un filetage de vis hélicoïdale à pas inverse du précédent (désigné plus loin par "CF" avec une valeur de pas négative) qui repousse la matière traitée dans le sens allant de la sortie vers l'entrée du mélangeur, un tel filetage comportant des rainures longitudinales pour assurer le passage de la matière vers la sortie du mélangeur, et enfin, un tronçon multilobé comportant sur toute sa longueur des palettes (ou lobes) disposées côte à côte et angulairement décalées les unes par rapport aux autres. Un tronçon bilobé comporte donc une succession de lobes décalés de 90° les uns par rapport aux autres et est désigné ci-après par "BL". On peut disposer d'un assez grand nombre de manchons à filetage externe pour faire varier le pas, la profondeur et le nombre de filets dans les différentes zones longitudinales successives du mélangeur. De plus, les différentes zones longitudinales du mélangeur peuvent être chauffées par un ou plusieurs fourreaux disposés à l'extérieur de l'enveloppe externe. Le chauffage peut être effectué dans chaque fourreau à l'aide d'au moins une résistance électrique ou d'au moins un échangeur de chaleur.

Selon l'invention, l'arbre (ou chaque arbre) du mélangeur extrudeur-cuiseur comporte, de préférence, au moins un manchon "DF" formant une vis de transport situé du côté de l'alimentation (ou entrée) du mélangeur, au moins un manchon "CF" (dit "à contrefilet") et/ou un manchon "BL" multilobes constituant une zone de malaxage sous pression, et au moins un manchon "DF" formant une vis de transport situé à l'extrémité de sortie du mélangeur. L'appareil peut également ment comporter au moins un manchon ayant une action de broyage et/ou d'homogénéisation tels qu'un manchon bilobé "BL".

Le procédé selon la présente invention a l'avantage d'être flexible, car on peut facilement faire varier les produits introduits en alimentation ainsi que les débits d'alimentation et, par conséquent, les formulations. On peut facilement également, en fonction de la composition de maquillage désirée, faire varier les paramètres physiques du traitement tels que la pression, notamment par action sur la section de sortie, la vitesse de rotation des arbres, le cisaillement en cours de traitement, notamment par le choix des manchons multilobes et des formes de filets, le malaxage, notamment par le choix des manchons de type "CF", et la température par régulation des fourreaux thermiques au droit des différentes zones du mélangeur.

La présente invention concerne également un produit de maquillage constitué de la composition selon l'invention telle qu'extrudée par mise en oeuvre du procédé ci-dessus décrit. L'invention concerne aussi un produit de maquillage constitué de la composition selon l'invention ayant subi, après extrusion selon le procédé ci-dessus défini, une opération de moulage ou de compactage mécanique, éventuellement suivie d'un séchage à l'air ou en étuve.

L'invention concerne enfin un produit de maquillage constitué d'un gel contenant, dispersé à l'état particulaire, un extrudat obtenu par mise en oeuvre du procédé ci-dessus défini, ledit extrudat ayant subi une granulation pour constituer les particules introduites dans ledit gel.

Les exemples donnés ci-après, à titre illustratif et non limitatif, permettront de mieux comprendre l'invention.

EXEMPLE 1

a) Mélangeur extrudeur utilisé

On opère dans un mélangeur extrudeur-cuiseur à deux vis (type BC 21 de la société "CLEXTRAL"), dont la structure est celle schématisée ci-dessous :

| Entrée --- > | | | | | | | | --- > Sortie |
|---|---|---|---|---|---|---|---|---|
| Structure de vis | DF | DF | DF | CF | DF | DF | DF | BL | DF |
| Longueur des manchons (mm) | 100 | 100 | 75 | 25 | 75 | 100 | 50 | 50 | 25 |
| Longueur des pas de vis (mm) | 33 | 25 | 16,6 | -16,6 | 16,6 | 25 | 16,6 | - | 16,6 |

Sur le dessin annexé :

- les figures 1, 3 et 5 représentent en élévation des tronçons de différents types de manchons utilisés sur les arbres du mélangeur mis en oeuvre dans l'exemple 1 ;
- les figures 2, 4 et 6 représentent respectivement des coupes transversales selon II-II, IV-IV et VI-VI des figures 1, 3 et 5.

En se référant au dessin, on voit que l'on a désigné par 1 l'enveloppe externe du mélangeur et par 2a, 2b les axes des deux arbres parallèles, qui y sont disposés. Sur les axes 2a, 2b sont enfilés des manchons adjacents, les deux arbres étant équipés des mêmes manchons sur un même tronçon de la longueur pour coopérer mécaniquement entre eux au cours de la rotation.

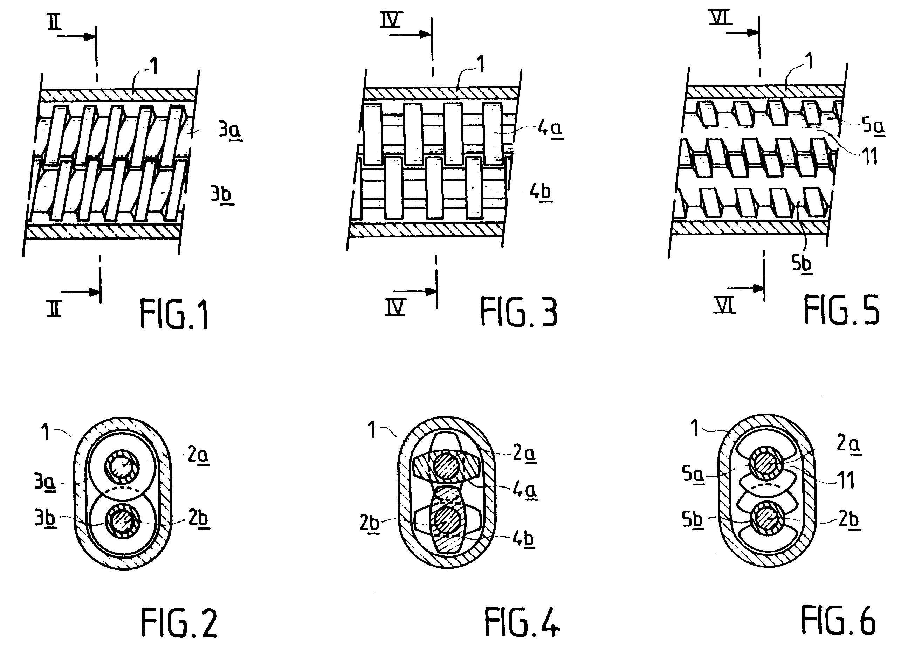

Sur les figures 1 et 2, on a représenté un tronçon où se trouvent des manchons de type "DF" référencés 3a, 3b. Sur les figures 3 et 4, on a représenté un tronçon où se trouvent des manchons 4a, 4b de type bilobé "BL". Sur les figures S et 6, on a représenté un tronçon où se trouvent des manchons 5a, 5b de type "CF" avec des rainures longitudinales 11.

Dans le tableau ci-dessus donné :

- DF représente un élément de vis à double filet hélicoïdal, tel qu'illustré sur les figures 1 et 2 ;
- BL représente un élément bilobé, tel qu'illustré sur les figures 3 et 4 ; et
- CF représente un élément de vis à pas inverse de DF, tel qu'illustré sur les figures 5 et 6, comportant des rainures longitudinales 11.

Les différents éléments ont un diamètre externe de 25 mm, un diamètre intérieur de 14 mm ; la distance entre les axes des deux arbres est de 21 mm.

Les deux arbres tournent à la vitesse de 300 t/min ; les orifices de sortie ont une section totale de 500 mm$^2$ ; le débit est de 5 kg/h environ.

Entre la tête d'arbre et la filière d'extrusion, le mélangeur ci-dessus décrit a une longueur de 600 mm. Son enveloppe extérieure est disposée à l'intérieur de six fourreaux adjacents (non représentés sur le dessin) où circulent des fluides thermostatés, chaque fourreau occupant une longueur de 100 mm. Le liquide circulant dans les deux fourreaux voisins de la tête d'arbre est à 80° C, celui circulant dans les deux fourreaux voisins de la filière d'extrusion est à 20° C

8

et celui circulant dans les deux fourreaux intermédiaires est à 60° C. Pour les exemples qui suivent, la fraction pulvérulente est introduite à l'aide d'un doseur pondéral en tête d'arbre et, éventuellement, partiellement à 100 mm de la filière d'extrusion. La phase grasse est introduite à l'aide d'une pompe péristaltique en tête d'arbre. La phase aqueuse est introduite à l'aide d'une pompe péristaltique à 100 mm de la tête d'arbre. Cependant, si les phases de l'émulsion contiennent des constituants thermosensibles tels qu'une silicone volatile ou certains conservateurs, on peut introduire ces constituants à 100 mm de la filière d'extrusion. Il est du ressort de l'homme de métier, pour chaque composition spécifique, de réguler la température et de définir les points d'introduction pour obtenir la qualité cosmétique optimale de la composition selon l'invention.

B-Formulation

On réalise un fond de teint poudré estompant le relief de la peau.
La formulation est la suivante (poids donnés en grammes) :

| - Oxyde de fer jaune | 1,04 |
|---|---|
| - Oxyde de fer rouge-jaune | 0,52 |
| - Oxyde de fer noir | 0,14 |
| - Dioxyde de titane | 6,3 |
| - Triéthanolamine | 0,69 |
| - Acide stéarique | 1,38 |
| - Glycéryl stéarate | 1,38 |
| - Conservateur | 0,1 |
| - Isoparaffine | 4,41 |
| - Microsphères expansées vendues par la société "KEMANORD PLAST" sous la dénomination commerciale "EXPANCEL" | 0,59 |
| - Microsphères de silice vendues par la société "MIYOSHI" sous la dénomination commerciale "SILICABEADS SB 700" | 44,41 |
| - Talc | 5 |
| - Eau      qsp | 100 |

Pour cette formulation, la densité D de la fraction pulvérulente est de 0,4656 (la valeur correspondante de T0 est 31,9 %) ; le taux de charges T est de 50 % ; la valeur de C est d'environ 90 % et la valeur de C* est de 33,75 %.
On obtient un fond de teint poudré applicable sur le visage au moyen d'une éponge en latex ; une application uniforme d'environ 1 gramme pour l'ensemble du visage conduit à un maquillage estompant le relief de la peau mais conservant un aspect naturel.

EXEMPLE 2 : Préparation d'un fond de teint compacté

On opère dans le même mélangeur extrudeur-cuiseur que pour l'exemple 1 avec la même structure de vis, les mêmes températures, les mêmes vitesses d'arbres et les mêmes orifices de sortie.
La formulation de la composition est la suivante (poids donnés en grammes) :

| - Oxyde de fer jaune | 0,55 |
|---|---|
| - Oxyde de fer rouge-jaune | 0,55 |
| - Oxyde de fer noir | 0,25 |
| - Dioxyde de titane | 3,65 |

(suite)

| | |
|---|---|
| - Triéthanolamine | 1,1 |
| - Acide stéarique | 2,2 |
| - Glycéryl stéarate | 2,2 |
| - Conservateur | 0,3 |
| - Isoparaffine | 5 |
| - Microsphères expansées vendues par la société "KEMANORD PLAST" sous la dénomination commerciale "EXPANCEL" | 2 |
| - Microsphères de silice vendues par la société "MIYOSHI" sous la dénomination commerciale "SILICABEADS SB 150" | 30 |
| - Microsphères de polyméthacrylate de méthyle vendues par la société "SEPPIC" sous la dénomination commerciale "MICROPEARL" | 8 |
| - Eau | 42,20 |

Pour cette formulation, la densité D de la fraction pulvérulente est de 0,6465 (ce qui correspond à une valeur T0 = 40 %) ; le taux de charges T est de 40 % ; la valeur de C est d'environ 85 % et la valeur de C* est de 46,9 %.

Le produit extrudé obtenu est mis en forme par compactage dans une presse mécanique puis laissé à l'air libre. Le produit de maquillage compacté ne présente au cours du temps aucun signe de craquelure. Cette poudre appliquée sur un visage, à raison de 1 gramme pour un visage, a un très bon pouvoir couvrant et donne un maquillage d'aspect naturel.

EXEMPLE 3 : Fond de teint poudré compacté

On opère dans le même mélangeur que pour l'exemple 1 avec les mêmes conditions de fonctionnement du mélangeur.

La formulation de la composition est la suivante (poids donnés en grammes) :

| | |
|---|---|
| - Oxyde de fer jaune | 0,55 |
| - Oxyde de fer rouge-jaune | 0,55 |
| - Oxyde de fer noir | 0,25 |
| - Dioxyde de titane | 3,65 |
| - Triéthanolamine | 1,1 |
| - Acide stéarique | 2,2 |
| - Glycéryl stéarate | 2,2 |
| - Conservateur | 0,3 |
| - Isoparaffine | 5 |
| - Talc | 20 |
| - Microsphères de silice vendues par la société "MIYOSHI" sous la dénomination commerciale "SILICABEADS SB 150" | 30 |
| - Glycérine | 5 |
| - Eau | 29,20 |

Pour cette composition, la densité D de la fraction pulvérulente est de 2,44 (ce qui correspond à une valeur T0 = 40 %) ; le taux de charges T est de 50 % ; la valeur de C est d'environ 85 % et la valeur de C* est de 34,40 %.

Le produit extrudé obtenu est compacté dans une presse mécanique puis laissé à l'air libre : on ne constate aucune craquelure au cours du temps. Ce produit constitue un fond de teint applicable sur le visage ; à raison de 1 gramme par visage, on constate que le fond de teint a un très bon pouvoir couvrant et qu'il permet d'obtenir un maquillage homogène d'aspect naturel.

EXEMPLE 4 : Gel de maquillage

On réalise un gel de maquillage en utilisant le fond de teint obtenu à l'exemple 1 ; ce fond de teint est mis sous forme de granules à la sortie du mélangeur extrudeur grâce à un granulateur branché directement sur l'extrudeur. Les granulés sont incorporés dans un gel constitué de 95 % d'eau et 5 % de carboxyméthylcellulose. On incorpore 20 % en poids de granulés dans ce gel ; les granulés ont des dimensions moyennes d'environ 1 mm.

Lorsqu'on utilise ce gel pour le maquillage d'un visage, on constate que l'application donne une sensation de fraicheur et, après séchage, il reste une fine pellicule de poudre, qui donne un teint très naturel et estompe les rides.

EXEMPLE 5

On utilise le fond de teint de l'exemple 1 pour constituer par moulage un bâton (ou "stick") de maquillage. Le "stick" de maquillage est conservé sous conditionnement étanche ; il permet une application de fond de teint, qui donne un très bon estompage des reliefs de la peau.

**Revendications**

1. Composition cosmétique destinée au maquillage de la peau comprenant une fraction pulvérulente de densité D compatible avec l'application sur la peau, dispersée dans un liant formé par une émulsion comportant une phase grasse et une phase aqueuse, dans laquelle ;

   - les particules de ladite fraction ont des dimensions comprises entre 0,5 et 100 µm,
   - la concentration particulaire volumique de ladite composition est au moins égale à la concentration particulaire critique C*, et
   - le taux de charges T, défini en % par la formule :

$$T = 100 \times \frac{\text{poids de la fraction pulvérulente hors pigments}}{\text{poids total de la composition}}$$

   est compris entre T0 et 1,8 x T0, la valeur T0 étant calculée de la manière suivante :

   - si D ≤ 0,1        T0 = 10
   - si 0,1 < D ≤ 0,6        T0 = (60 x D) + 4
   - si D > 0,6        T0 = 40

2. Composition selon la revendication 1, caractérisée par le fait que C* est compris entre 10 et 80 %.

3. Composition selon l'une des revendications 1 ou 2, caractérisée par le fait que C (concentration particulaire volumique) est supérieur à 80 %.

4. Composition selon l'une des revendications 1 à 3, caractérisée par le fait que, si la fraction pulvérulente a une densité supérieure ou égale à 0,6, T est compris entre 40 % et 65 %.

5. Composition selon l'une des revendications 1 à 4, caractérisée par le fait que la fraction pulvérulente comporte au moins un pigment et/ou au moins une charge.

6. Composition selon la revendication 5, caractérisée par le fait que la fraction pulvérulente comprend au moins une charge incompactable.

7. Composition selon la revendication 5, caractérisée par le fait que le(s) pigment(s) est (sont) choisi(s) dans le groupe formé par les pigments minéraux, les pigments organiques, les pigments nacrés blancs et/ou colorés.

8. Composition selon la revendication 5, caractérisée par le fait que la (les) charge(s) est (sont) choisie(s) dans le groupe formé par le talc, les micas, les micas-titanes, les séricites, l'amidon, le kaolin, les oxydes de zinc et de titane, le carbonate de calcium précipité, les savons métalliques dérivés d'acides organiques carboxyliques ayant de 8 à 22 atomes de carbone, les poudres de polymères synthétiques, la silice, les poudres organiques, les poudres de silicone, les poudres fluorées, les microsphères pleines, les microsphères pleines micro-poreuses, imprégnées ou non, les microcapsules polymériques, les microsphères creuses.

9. Composition selon l'une des revendications 1 à 8, caractérisée par le fait qu'elle contient de 25 à 50 % en poids, et, de préférence, de 30 à 45 % en poids, d'eau.

10. Composition selon l'une des revendications 1 à 9, caractérisée par le fait que la phase grasse comprend au moins un constituant choisi dans le groupe formé par les huiles ou corps gras d'origine végétale, animale, minérale ou synthétique et les cires d'origine végétale, animale, minérale ou synthétique.

11. Composition selon l'une des revendications 1 à 10, caractérisée par le fait que la phase grasse contient au moins un ingrédient oléosoluble et/ou un actif cosmétique lipophile, l'ensemble de ces additifs représentant au plus 20 % en poids par rapport au poids total de la phase grasse.

12. Composition selon l'une des revendications 1 à 11, caractérisée par le fait que la phase grasse contient des huiles volatiles ayant à 25° C une tension de vapeur saturante supérieure à 50 pascals, ces huiles représentant moins de 10 % en poids par rapport au poids total de la composition et moins de 20 % en poids par rapport au poids de la phase grasse.

13. Composition selon l'une des revendications 1 à 12, caractérisée par le fait que la phase aqueuse de l'émulsion contient, en plus de l'eau, au moins un additif pris dans le groupe formé par des épaississants, des humectants, des hydratants ou des adjuvants hydrosolubles généralement utilisés en cosmétique, ces additifs représentant au plus 40 % en poids par rapport au poids total de la composition.

14. Procédé de fabrication d'une composition selon l'une des revendications 1 à 13, caractérisé par le fait que l'on effectue le mélange des divers constituants de ladite composition dans un mélangeur extrudeur-cuiseur comportant, dans une enveloppe externe (1) munie en sortie d'une filière d'extrusion, un (ou deux) arbre(s) entraîné(s) en rotation de façon que la structure périphérique d'un arbre coopère avec l'enveloppe externe (1) et, le cas échéant, avec la structure périphérique de l'autre arbre pour assurer un mélangeage de la matière et son déplacement dans l'enveloppe externe (1) vers la filière d'extrusion.

15. Procédé selon la revendication 14, caractérisé par le fait que l'(les) arbre(s) est (sont) constitué(s) d'au moins deux manchons successifs (3a, 3b ; 4a, 4b ; 5a, 5b), dont la partie intérieure s'adapte sur un axe (2a, 2b) entraîné en rotation et la partie extérieure a une structure périphérique différente selon les manchons.

16. Procédé selon l'une des revendications 14 ou 15, caractérisé par le fait que l'arbre (ou chaque arbre) comporte au moins un manchon (3a ou 3b) formant une vis de transport situé du côté de l'alimentation du mélangeur, au moins un manchon (5a ou 5b) à contrefilet et/ou un manchon (4a, 4b) multilobes, et un moins un manchon (3a ou 3b) formant une vis de transport situé à l'extrémité de sortie du mélangeur.

17. Procédé selon l'une des revendications 14 à 16, caractérisé par le fait que l'on introduit dans le mélangeur, simultanément mais en des points décalés le long de l'(ou des) arbre(s), la phase grasse du liant, la phase aqueuse du liant et la fraction pulvérulente, la température allant en décroissant depuis la tête d'arbre jusqu'à la filière d'extrusion.

18. Procédé selon la revendication 17, caractérisé par le fait que la température est d'environ 70-90° C au voisinage de la tête d'arbre et décroît jusqu'à 20-30° C au voisinage de la filière d'extrusion.

19. Procédé selon la revendication 18, caractérisé par le fait que les constituants non-thermosensibles volatils de la phase grasse sont introduits au voisinage de la tête d'arbre, de même que tout ou partie de la fraction pulvérulente, les constituants non-thermosensibles de la phase aqueuse sont introduits en un point plus éloigné de la tête d'arbre et les constituants thermosensibles volatils de l'émulsion ainsi que le reste éventuel de la fraction pulvérulente sont introduits en un point encore plus éloigné de la tête d'arbre.

20. Produit de maquillage constitué de la composition selon l'une des revendications 1 à 13 telle qu'extrudée par mise en oeuvre du procédé selon l'une des revendications 14 à 19.

21. Produit de maquillage constitué de la composition selon l'une des revendications 1 à 13, ladite composition ayant subi après extrusion selon le procédé de l'une des revendications 14 à 19, une opération de moulage ou de compactage mécanique, éventuellement suivie d'un séchage à l'air ou en étuve.

22. Produit de maquillage constitué d'un gel contenant, dispersé à l'état particulaire, un extrudat obtenu par mise en oeuvre du procédé selon l'une des revendications 14 à 19, ledit extrudat ayant subi une granulation pour constituer les particules introduites dans ledit gel.

## Claims

1. Cosmetic composition intended for making up the skin, including a pulverent fraction of relative density D, compatible with application to the skin, dispersed in a binder consisting of an emulsion comprising a fatty phase and an aqueous phase, in which:

    - the particles of the said fraction have dimensions of between 0.5 and 100 $\mu$m,
    - the particle concentration per unit volume of the said composition is at least equal to the critical particle concentration $C^*$, and
    - the filler content F, defined in % by the formula:

    $$F = 100 \times \frac{\text{weight of the pulverulent fraction excluding pigments}}{\text{total weight of the composition}}$$

    is between F0 and 1.8 x F0, the value F0 being calculated as follows:

    - if $D \leq 0.1$        F0 = 10
    - if $0.1 < D \leq 0.6$        F0 = $(60 \times D) + 4$
    - if $D > 0.6$        F0 = 40

2. Composition according to Claim 1, characterized in that $C^*$ is between 10 and 80 %.

3. Composition according to either of Claims 1 and 2, characterized in that C (particle concentration per unit volume) is greater than 80 %.

4. Composition according to one of Claims 1 to 3, characterized in that, if the pulverulent fraction has a relative density higher than or equal to 0.6, F is between 40 % and 65 %.

5. Composition according to one of Claims 1 to 4, characterized in that the pulverulent fraction comprises at least one pigment and/or at least one filler.

6. Composition according to claim 5, characterized in that the pulverulent fraction includes at least one noncompactable filler.

7. Composition according to claim 5, characterized in that the pigment(s) is (are) chosen from the group consisting of inorganic pigments, organic pigments and white and coloured pearlescent pigments.

8. Composition according to Claim 5, characterized in that the filler(s) is (are) chosen from the group consisting of talc, micas, titanium-micas, sericites, starch, kaolin, zinc and titanium oxides, precipitated calcium carbonate, metal soaps derived from carboxylic organic acids containing from 8 to 22 carbon atoms, synthetic polymer powders, silica, organic powders, silicone powders, fluorinated powders, solid microspheres, microporous solid microspheres which may be unimpregnated or impregnated, polymer microcapsules and hollow microspheres.

9. Composition according to one of Claims 1 to 8, characterized in that it contains from 25 to 50 % by weight and preferably from 30 to 45 % by weight of water.

10. Composition according to one of Claims 1 to 9, characterized in that the fatty phase includes at least one constituent chosen from the group consisting of fatty oils or substances of plant, animal, mineral or synthetic origin and waxes of vegetable, animal, mineral or synthetic origin.

11. Composition according to one of Claims 1 to 10, characterized in that the fatty phase contains at least one oil-soluble ingredient and/or a lipophilic cosmetic agent, the combination of these additives representing not more than 20 % by weight relative to the total weight of the fatty phase.

12. Composition according to one of Claims 1 to 11, characterized in that the fatty phase contains volatile oils which at 25°C have a saturated vapour pressure higher than 50 pascals, these oils representing less than 10 % by weight relative to the total weight of the composition and less than 20 % by weight relative to the weight of the fatty phase.

13. Composition according to one of Claims 1 to 12, characterized in that the aqueous phase of the emulsion contains, in addition to the water, at least one additive taken from the group consisting of thickeners, moisturizers, hydrating agents or water-soluble adjuvants generally employed in cosmetics, these additives representing not more than 40 % by weight relative to the total weight of the composition.

14. Process for the manufacture of a composition according to one of Claims 1 to 13, characterized in that the mixing of the various constituents of the said composition is performed in a cooker-extruder mixer comprising, in an outer casing (1) provided with an extrusion die at the exit, one (or two) shaft(s) driven in rotation so that the peripheral structure of a shaft interacts with the outer casing (1) and, where appropriate, with the peripheral structure of the other shaft, to ensure blending of the material and its movement in the outer casing (1) towards the extrusion die.

15. Process according to Claim 14, characterized in that the shaft(s) consist(s) of at least two successive sleeves (3a, 3b; 4a, 4b; 5a, 5b) whose inner part is fitted onto an axle (2a, 2b) driven in rotation, and the outer part has a peripheral structure which differs according to the sleeves.

16. Process according to either of Claims 14 and 15, characterized in that the shaft (or each shaft) comprises at least one sleeve (3a or 3b) forming a conveying screw situated on the feed side of the mixer, at least one sleeve (5a or 5b) with reverse flight and/or a multilobar sleeve (4a, 4b) and at least one sleeve (3a or 3b) forming a conveying screw situated at the exit end of the mixer.

17. Process according to one of Claims 14 to 16, characterized in that the fatty phase of the binder, the aqueous phase of the binder and the pulvervlent fraction are introduced into the mixer simultaneously but at points offset along the shaft(s), with the temperature decreasing from the head of the shaft to the extrusion die.

18. Process according to Claim 17, characterized in that the temperature is approximately 70-90°C in the vicinity of the head of the shaft and decreases to 20-30°C in the vicinity of the extrusion die.

19. Process according to Claim 18, characterized in that the volatile constituents of the fatty phase which are not heat-sensitive are introduced in the vicinity of the head of the shaft, as is all or part of the pulverulent fraction, the constituents of the aqueous phase which are not heat-sensitive are introduced at a point further away from the head of the shaft and the volatile heat-sensitive constituents of the emulsion, together with any remainder of the pulverulent fraction are introduced at a point which is still further away from the head of the shaft.

20. Make-up product consisting of the composition according to one of Claims 1 to 13, as extruded by making use of the process according to one of Claims 14 to 19.

21. Make-up product consisting of the composition according to one of Claims 1 to 13, the said composition having undergone, after extrusion according to the process of one of Claims 14 to 19, a mechanical moulding or compacting operation, optionally followed by drying in air or in an oven.

22. Make-up product consisting of a gel containing, dispersed in the particulate state, an extrudate obtained by making use of the process according to one of Claims 14 to 19, the said extrudate having undergone a granulation to form the particles introduced into the said gel.

**Patentansprüche**

1. Kosmetische Zusammensetzung zum Schminken der Haut, umfassend eine hautanwendungsverträgliche pulverförmige Fraktion mit der Dichte D, die in einem Bindemittel dispergiert ist, das aus einer Emulsion mit einer Fettphase und einer wäßrigen Phase gebildet wird, in der

- die Partikel der Fraktion Abmessungen zwischen 0,5 und 100 μm aufweisen,
- die Partikelvolumenkonzentration der Zusammensetzung wenigstens der kritischen Partikelkonzentration C* entspricht und
- der Anteil an Füllstoffen T, ausgedrückt in % durch die Formel:

$$T = 100 \times \frac{\text{Gewicht der pulverförmigen Fraktion ohne Pigmente}}{\text{Gesamtgewicht der Zusammensetzung}}$$

T0 bis 1,8 x T0 beträgt, wobei der Wert T0 folgendermaßen berechnet wird:

- falls $D \leq 0,1$     T0 = 10
- falls $0,1 < D \leq 0,6$     T0 = (60 x D) + 4
- falls $D > 0,6$     T0 = 40

2.  Zusammensetzung nach Anspruch 1, dadurch gekennzeichnet, daß C* 10 bis 80 % beträgt.

3.  Zusammensetzung nach einem der Ansprüche 1 oder 2, dadurch gekennzeichnet, daß C (Partikelvolumenkonzentration) größer als 80 % ist.

4.  Zusammensetzung nach einem der Ansprüche 1 bis 3, dadurch gekennzeichnet, daß T 40 % bis 65 % beträgt, falls die pulverförmige Fraktion eine Dichte von wenigstens 0,6 aufweist.

5.  Zusammensetzung nach einem der Ansprüche 1 bis 4, dadurch gekennzeichnet, daß die pulverförmige Fraktion wenigstens ein Pigment und/oder wenigstens einen Füllstoff aufweist.

6.  Zusammensetzung nach Anspruch 5, dadurch gekennzeichnet, daß die pulverförmige Fraktion einen nichtkompaktierbaren Füllstoff aufweist.

7.  Zusammensetzung nach Anspruch 5, dadurch gekennzeichnet, daß das Pigment (die Pigmente) ausgewählt ist (sind) unter mineralischen Pigmenten, organischen Pigmenten, weißen und/oder farbigen perlmuttartigen Pigmenten.

8.  Zusammensetzung nach Anspruch 5, dadurch gekennzeichnet, daß der Füllstoff (die Füllstoffe) ausgewählt ist (sind) unter Talg, Glimmern, Titanglimmern, Sericiten, Stärke, Kaolin, Zink- und Titanoxiden, gefälltem Calciumcarbonat, Metallseifen organischer Carbonsäuren mit 8 bis 22 Kohlenstoffatomen, synthetischen Polymerpulvern, Siliciumdioxid, organischen Pulvern, Silikonpulvern, fluorierten Pulvern, ausgefüllten Mikrosphären, gegebenenfalls imprägnierten mikroporösen ausgefüllten Mikrosphären, Polymermikrokapseln, hohlen Mikrosphären.

9.  Zusammensetzung nach einem der Ansprüche 1 bis 8, dadurch gekennzeichnet, daß sie 25 bis 50 Gew.-% und vorzugsweise 30 bis 45 Gew.-% Wasser enthält.

10. Zusammensetzung nach einem der Ansprüche 1 bis 9, dadurch gekennzeichnet, daß die Fettphase wenigstens einen Bestandteil aufweist, der ausgewählt ist unter Ölen oder Fettkörpern pflanzlichen, tierischen, mineralischen oder synthetischen Ursprungs und Wachsen pflanzlichen, tierischen, mineralischen oder synthetischen Ursprungs.

11. Zusammensetzung nach einem der Ansprüche 1 bis 10, dadurch gekennzeichnet, daß die Fettphase wenigstens einen öllöslichen Inhaltsstoff und/oder einen lipophilen kosmetischen Wirkstoff enthält, wobei die Gesamtheit dieser Additive höchstens 20 Gew.-% des Gesamtgewichts der Fettphase ausmacht.

12. Zusammensetzung nach einem der Ansprüche 1 bis 11, dadurch gekennzeichnet, daß die Fettphase flüchtige Öle mit einem Sättigungsdampfdruck von mehr als 50 Pascal bei 25° C enthält, wobei diese Öle wenigstens 10 Gew.-% des Gesamtgewichts der Zusammensetzung und wenigstens 20 Gew.-% des Gewichts der Fettphase ausmachen.

13. Zusammensetzung nach einem der Ansprüche 1 bis 12, dadurch gekennzeichnet, daß die wäßrige Phase der Emulsion zusätzlich zum Wasser wenigstens ein Additiv enthält, das ausgewählt ist unter Verdickungsmitteln, Netzmitteln, Hydratisierungsmitteln oder wasserlöslichen Adjuvantien, die üblicherweise in der Kosmetik verwendet werden, wobei diese Additive höchstens 40 Gew.-% des Gesamtgewichts der Zusammensetzung ausmachen.

14. Verfahren zur Herstellung einer Zusammensetzung nach einem der Ansprüche 1 bis 13, dadurch gekennzeichnet, daß man die verschiedenen Bestandteile der Zusammensetzung in einem beheizbaren Extrusionsmischer vermischt, der in einem am Ausgang mit einem Extrusionswerkzeug versehenem Außenmantel (1) eine (oder zwei) Welle(n) aufweist, die so in Rotation versetzt wird (werden), daß die periphäre Struktur einer Welle mit dem Außenmantel (1) und gegebenenfalls mit der periphären Struktur der anderen Welle zusammenarbeitet, um ein Vermischen des Stoffes und dessen Bewegung im Außenmantel (1) in Richtung auf das Extrusionswerkzeug zu gewährleisten.

15. Verfahren nach Anspruch 14, dadurch gekennzeichnet, daß die Welle(n) aus wenigstens zwei aufeinanderfolgenden Stegen (3a, 3b; 4a, 4b; 5a, 5b) gebildet wird (werden), deren Innenteil an eine in Rotation versetzte Achse (2a, 2b) angefügt ist und deren Außenteil eine je nach Steg unterschiedliche periphäre Struktur aufweist.

16. Verfahren nach einem der Ansprüche 14 oder 15, dadurch gekennzeichnet, daß die Welle (oder jede Welle) wenigstens einen Steg (3a oder 3b), welcher eine auf der Seite der Vorrichtungsbeschickung gelegene Transportschraube bildet, wenigstens einen gegengängigen Steg (5a oder 5b) und/oder mehrlappigen Steg (4a, 4b), und wenigstens einen Steg (3a oder 3b), welcher eine am Ende des Mischvorrichtungsausganges gelegene Transportschraube bildet, aufweist.

17. Verfahren nach einem der Ansprüche 14 bis 16, dadurch gekennzeichnet, daß man gleichzeitig aber an entlang der Welle (oder der Wellen) versetzten Punkten die Fettphase des Bindemittels, die wäßrige Phase des Bindemittels und die pulverförmige Fraktion in die Mischvorrichtung einbringt, wobei die Temperatur vom Wellenkopf bis zum Extrusionswerkzeug abnimmt.

18. Verfahren nach Anspruch 17, dadurch gekennzeichnet, daß die Temperatur etwa 70 bis 90° C in der Nähe des Wellenkopfes beträgt und auf 20 bis 30° C in der Nähe des Extrusionswerkzeuges abnimmt.

19. Verfahren nach Anspruch 18, dadurch gekennzeichnet, daß die flüchtigen wärmeunempfindlichen Bestandteile der Fettphase genauso wie die gesamte oder ein Teil der pulverförmigen Fraktion in der Nähe des Wellenkopfes eingebracht werden, die wärmeunempfindlichen Bestandteile der wäßrigen Phase an einem vom Wellenkopf entfernteren Punkt eingebracht werden und die flüchtigen wärmeempfindlichen Bestandteile der Emulsion und auch eventuelle Reste der pulverförmigen Fraktion an einem vom Wellenkopf noch entfernteren Punkt eingebracht werden.

20. Schminkprodukt mit der Zusammensetzung aus einem der Ansprüche 1 bis 13, erhältlich durch Extrusion nach dem Verfahren aus einem der Ansprüche 14 bis 19.

21. Schminkprodukt mit der Zusammensetzung aus einem der Ansprüche 1 bis 13, wobei die Zusammensetzung im Anschluß an die Extrusion nach dem Verfahren aus einem der Ansprüche 14 bis 19 einer Formgebungs- oder mechanischen Kompaktierungsmaßnahme unterworfen und gegebenenfalls anschließend luft- oder ofengetrocknet wird.

22. Schminkprodukt aus einem Gel, das ein partikelförmiges dispergiertes Extrudat enthält, welches nach dem Verfahren aus einem der Ansprüche 14 bis 19 erhalten wird, wobei das Extrudat einer Granulation unterworfen wird, um die in das Gel eingebrachten Partikel zu bilden.

FIG. 1
FIG. 2
FIG. 3
FIG. 4
FIG. 5
FIG. 6